# EUROPEAN PATENT SPECIFICATION

(11) **EP 1 280 528 B1**
(45) Date of publication and mention of the grant of the patent: **23.03.2005**
(21) Application number: 01937291.1
(22) Date of filing: 10.05.2001
(51) Int. Cl.: A61K 31/435, A61P 27/06

(54) **R-ELIPRODIL FOR TREATING GLAUCOMA**
R-ELIPRODIL ZUR BEHANDLUNG VON GLAUCOMA
R-ELIPRODIL POUR LE TRAITEMENT DU GLAUCOME

(30) Priority: 10.05.2000 US 203350 P
(43) Date of publication of application: 05.02.2003
(73) Proprietor: Alcon, Inc, 6331 Hünenberg (CH)
(72) Inventor: KAPIN, Michael, A., Arlington, TX 76016 (US); BERGAMINI, Michael, V. W., Burleson, TX 76028 (US); SALLEE, Verney, L., Burleson, TX 76028 (US)
(74) Representative: Wain, Christopher Paul
(86) International application number: PCT/US2001/015169
(87) International publication number: WO 2001/085169

(56) References cited:
- WO-A-00/04898
- WO-A-00/04899
- FR-A- 2 628 740
- US-A- 5 023 266
- US-A- 5 710 165
- US-A- 5 883 108
- VARIOUS: "the merck manual" 1999 , BEERS/BERKOW , N.J. XP002184733 page 737; table 1

## Description

The present invention is directed to the use of R-α-(4-chlorophenyl)-4-(4-fluorophenylmethyl)-1-peperidineethanol or its salts, hereinafter R-eliprodil, to treat glaucoma.

### Background of the Invention

Eliprodil, a racemic compound, is a systemically active neuroprotectant. Its structure and synthesis are disclosed in U.S. Patent No. 4,690,931. Eliprodil is also useful for the preservation of visual field in persons suffering from glaucoma, see U.S. Patent No. 5,710,165 and for treating ischemic disorders of the eye and optic nerve head, see U.S. Patent No. 6,020,352. It is also known for its usefulness in treating persons with diabetic retinopathy, see WO 99/25350. None of these patents recognize the use of the R-isomer of eliprodil for the treatment of ophthalmic conditions.

There are presently no neuroprotective agents approved for the treatment of glaucoma. Glaucoma is characterized by the development of visual field defects concomitant with inner retina and/or optic nerve head pathology. Ischemia or trauma may play a role in the neuronal cell death associated with these retinal neuropathies due, at least in part, to a process termed excitotoxicity.

Excitotoxicity has been extensively implicated in disorders of the retina inclusive of glaucoma. In glaucoma, the pathological changes of the optic nerve head (cupping, excavation, thinning of neural rim) are linked to the loss of retinal ganglion cells, toxicity that may be caused by glutamate. To date, numerous lines of evidence have demonstrated the sensitivity of cultured retinal ganglion cells to exogenously administered glutamate, perturbations of isolated or intact retinal tissue following application of a variety of excitatory amino acid (EAA) agonists, and distinct pathologies of retinal tissue following intravitreal injections of excitotoxins. Dreyer and coworkers have discussed a role for glutamate as a causative factor of glaucoma based upon the demonstration that patients with open-angle glaucoma or non-human primates with pressure-induced optic neuropathy have higher concentrations of glutamate in the vitreous. (Elevated glutamate levels in the vitreous body of humans and monkeys with glaucoma. Dreyer, et al., Arch. Ophthalmology, Vol. 14:299-300, 1996.)

It is well established that blockage of specific EAA receptors can impart resistance to neuronal tissue damage in the face of ischemia, trauma, or other metabolic disturbances. Numerous studies have shown protection of retinal tissue by selective EAA antagonists, particularly those that target the N-methyl-D-aspartate (NMDA) receptor. Thus, antagonists of the NMDA receptor are neuroprotective. In Scatton, et al., Drugs of the Future, Vol. 19(10):905-909, (1994), racemic eliprodil, a compound with potent NMDA antagonist activity, was shown to be neuroprotective in a variety of neurodegenerative disease models in the brain including cerebral ischemia and brain trauma. In Malavasi, et al., Journal of Chromatography A, Vol. 729:323-333, (1996), it was stated that both eliprodil and its enantiomers have comparable neuroprotective activity. However, in *an in vivo* model of rat focal cerebral ischemia, it was shown that the R-enantiomer, versus the S-enantiomer of eliprodil, contributes significantly to the *in vivo* biological activity of racemic eliprodil. (DiFabio, et al. Biorganic and Medicinal Chemistry Letters, Vol. 5(6):551-554, (1995). Although this study does demonstrate a difference in neuroprotective activity between the enantiomers, it is not a glaucoma model, nor is it a model for assessing NMDA activity since a variety of cellular mechanisms have been cited for the observed cerebral pathology. It should be noted that not all antagonists of diversely distributed EAA receptors would be suitable for use as neuroprotectants of the inner retina for a variety of reasons. For example, MK801 would not be useful as it has psychotomimetic properties.

Side effects always present a significant barrier to the development of a drug. One such effect involves the disturbance of cardiac repolarization. A number of pharmacological agents act to prolong cardiac repolarization by inhibiting membrane potassium channels of cardiomyocytes. In addition, some individuals have genetic defects of these membrane potassium channels with resulting prolongation of cardiac repolarization even in the absence of drugs. The prolongation of repolarization may be detected and/or quantitated by analysis of the electrocardiogram, using the parameter known as the QT interval. This parameter describes the time duration from onset of cardiac depolarization (indicated by the QRS wave) until the end of cardiac repolarization (indicated by the end of the T wave). A normal range for the QT interval is approximately 0.30 to 0.50 seconds. It is common to correct the QT interval for heart rate (identified as QTc, and normalized to 60 bpm heart rate), with a resulting normal range of values of 0.38 to 0.45 seconds.

Regardless of the initiating cause of prolongation of cardiac repolarization (QTc prolongation), this phenomenon may result in cardiac arrhythmia and possibly ventricular fibrillation leading to sudden death. A unique type of cardiac arrhythmia is often seen with QTc prolongation, termed *torsades de pointes,* that is readily identified with an electrocardiogram. The potential for sudden death clearly emphasizes the importance of characterizing the propensity for QTc prolongation prior to clinical use. It should be noted that drug combinations or the use of a single drug by a genetically susceptible person are of special concern.

Eliprodil (racemic) is known to produce asymptomatic QTc prolongation following some doses in humans. (Drugs of the Future, 1994, Vol. 19(10):905-909.)

### Summary of the Invention

The present invention is directed to the use of R-eliprodil to treat individuals suffering from glaucoma. The invention includes the treatment of glaucoma with R-eliprodil in combination with an additional agent for treating glaucoma.

### Brief Description of the Drawings

Figure 1 shows the protective effect in cultured retinal ganglion cells of R-eliprodil, S-eliprodil, and the racemate (RS).
Figure 2 compares the ability of RS-eliprodil, R-eliprodil, and memantine to improve recovery of synaptic transmission following a period of hypoxia.

### Detailed Description of Preferred Embodiments

Surprisingly, we have found that the R-enantiomer of eliprodil, rather than the S-enantiomer, is effective in preserving retinal ganglion cells from injury and thus, useful for treating persons suffering from glaucoma. Moreover, the R-enantiomer rather than the S-enantiomer or the racemate has less propensity to prolong QTc and thus, is safer for chronic use on humans.

Eliprodil, a neuroprotective agent which acts at the polyamine binding site of the NMDA receptor (NR2B subunit), has been shown to be potently neuroprotective in a variety of CNS trauma and ischemia models and, unlike many other NMDA antagonists, is devoid of CNS side-effects. In addition to activities at the NMDA site, eliprodil and its enantiomers are known to potently bind to sigma receptors in the rat brain (U.S. Patent No. 5,023,266.) Equipotency with the R-enantiomer and the S-enantiomer and the racemate has been observed regarding neurotrophic effects (U.S. Patent No. 5,547,963.)

We also know and disclose herein that eliprodil shows antagonism at voltage operated calcium channels of the L, N and P type, as well as sodium channels. Binding studies also demonstrate sub-micromolar affinity of eliprodil with histamine H₁, β₃-adrenergic, 5-HT uptake, 5-HT_{2A}, 5-HT_{1A}, histamine H₂, α₁-adrenergic, and 5-HT₇ binding sites. The role of these pharmacological activities in the neuroprotective efficacy has not been established for this drug.

We also know and disclose herein that eliprodil and its enantiomers (S(+) and R(-)) are neuroprotective in traumatic, ischemic, and excitotoxic models in the CNS, inclusive of the retina. Measured efficacy of the racemate/enantiomers *in vitro* is dependent on the pharmacology of the individual enantiomers as it relates to the applied insult. For example, electrophysiology and ligand displacement studies have shown that the racemate and R(-)-enantiomer possess significantly greater activity at the NMDA receptor than the S(+)-enantiomer (Kᵢ (nM) for R- and S-eliprodil at the NMDA receptor was 200 and 10,000, respectively). As shown in Example 1, marked neuroprotective activity was demonstrated against an NMDA insult by R-eliprodil and racemic eliprodil, but not S-eliprodil. Similarly, racemic, eliprodil or the R(-)-enantiomer was found *in vivo* to strongly inhibit NMDA-induced firing rate of the lateral vestibular nucleus, whereas the S(+)-enantiomer was almost inactive at the highest dose tested. As shown in Example 2, R-eliprodil provided for substantial recovery of excitatory post synaptic potentials following hypoxia in the hippocampal slice preparation. At a comparative dose, RS and S-eliprodil were less effective. The ability for eliprodil or the R(-)-enantiomer to inhibit excitatory amino acid mediated changes is important since excitotoxicity has been implicated in the underlying pathology of glaucoma. Neither enantiomer nor the racemate exhibited a marked neuroprotective advantage when tested in an *in vivo* model of cerebral ischemia (i.e. middle cerebral artery occlusion model in the rat) even though the R(-)-enantiomer and the S(+)-enantiomer have pharmacological differences. This finding is in contrast to that of DiFabio, et al., where the difference may represent the variety of mechanisms involved in cerebral pathology and differences in experimental models. Regardless, mechanisms other than or in addition to excitotoxicity are probably involved in this ischemic-induced neuronal degeneration. The importance of previously described neurotrophic effects as it relates to retinal degeneration is not known.

Despite significant affinity at a number of pharmacological binding sites, racemic eliprodil demonstrates no significant untoward effects on major organ systems other than the cardiovascular system. Eliprodil and its enantiomers were evaluated for their potential for prolonging QTc by determining their dose-related effects on cardiac electrical activity. Two different experimental models were utilized for these studies: (1) Action potential duration in piglet Purkinje fibers from the heart and (2) Electrocardiographic parameters in pentobarbital anesthetized cats. The results described below demonstrate that R(-) eliprodil has less propensity to prolong cardiac repolarization than either S(+) eliprodil or racemic eliprodil.

Eliprodil and its two enantiomers induced concentration-dependent prolongation of the duration of the action potential of the piglet Purkinje fibre, particularly at the 0.25 Hz frequency. Based on action potential duration at 90% repolarization (APD₉₀), statistically significant prolongation was evident at R(-) eliprodil concentrations of 100 nM and higher, but at S(+) eliprodil concentrations of 30 nM and higher. With high concentrations, APD₉₀ decreased with both compounds. For all concentrations, the maximum effect observed with R(-) eliprodil was less than that seen with S(+) eliprodil. Racemic eliprodil produced an effect intermediate between the two enantiomers for low concentrations, and achieved a maximum effect equal to that of S(+) eliprodil at high concentrations.

Eliprodil and its two enantiomers were also evaluated for hemodynamic and electrocardiographic parameters in pentobarbital-anesthetized cats. All three compounds induced hypotension, bradycardia, and a prolongation of QTc, although the dose response relationship differed among the compounds. Compounds were administered at increasing doses from 0.01 to 3 mg/kg as a five-minute intravenous infusion every 20 minutes. Threshold doses for each parameter are presented in Table 1. In the cat, QTc changes were evident at lower doses of each compound than either hypotension or bradycardia. The plasma level associated with a 0.1 mg/kg dose of R(-) eliprodil in this protocol was 34.9 ± 2.8 ng/ml.

**Table 1:**

| **Hemodynamic and Electrocardiographic Responses in the Anesthetized Cat** | | | |
|---|---|---|---|
| **Parameter** | **Threshold Dose (mg/kg)** | | |
| | R(-) Eliprodil | S(+) Eliprodil | Eliprodil |
| QTc Prolongation | 0.1 | 0.01 | 0.03 |
| Heart Rate Decrease | 0.3 | 0.1 | 0.1 |
| Mean Blood Pressure Decrease | 1.0 | 1.0 | 1.0 |

### EXAMPLE 1

### Neuroprotective Effect of Eliprodil and its Enantiomers on Glutamate-Induced Rat Retinal Ganglion Cell Death

Retinal ganglion cells were selected as they represent the retinal cell that degenerates in glaucoma. MK-801 was selected as a reference NMDA antagonist.

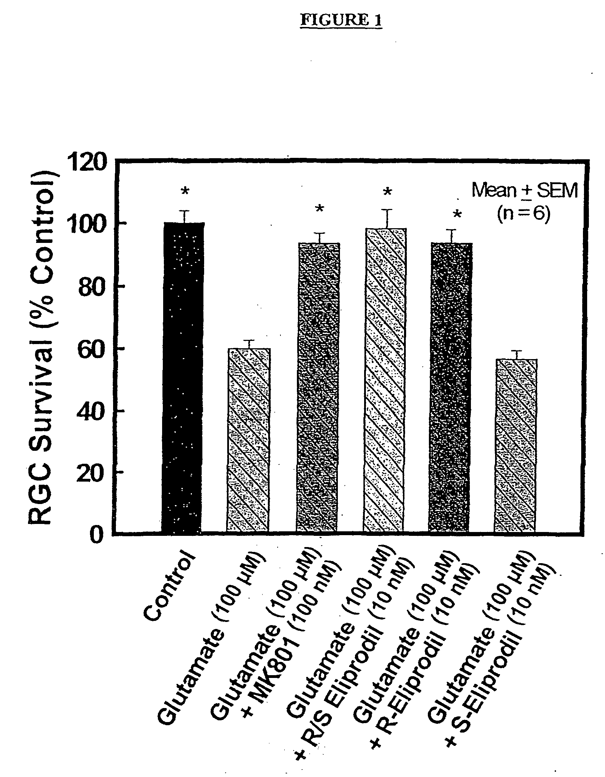

Isolation of rat retinal ganglion cells and assessment of viability in the absence and presence of glutamate was described in Pang, et al., "Protection by Eliprodil Against Excitotoxicity in Cultured Rat Retinal Ganglion Cells", *Investigative Ophthalmology & Visual Science,* Vol. 40(6), pp. 1170-1176, May, 1999. Briefly, isolated cultured rat retinal ganglion cells were treated with racemic (R/S) eliprodil (10nM), R-eliprodil (10nM), or S-eliprodil (10nM) for thirty (30) minutes followed by the addition of 100µM glutamate. Survival was determined by counting viable cells after an incubation period of three (3) days. Survival with vehicle alone defines 100%. As shown in Figure 1 R-eliprodil was as efficacious as a protectant as racemic eliprodil. S-eliprodil was ineffective.

### EXAMPLE 2

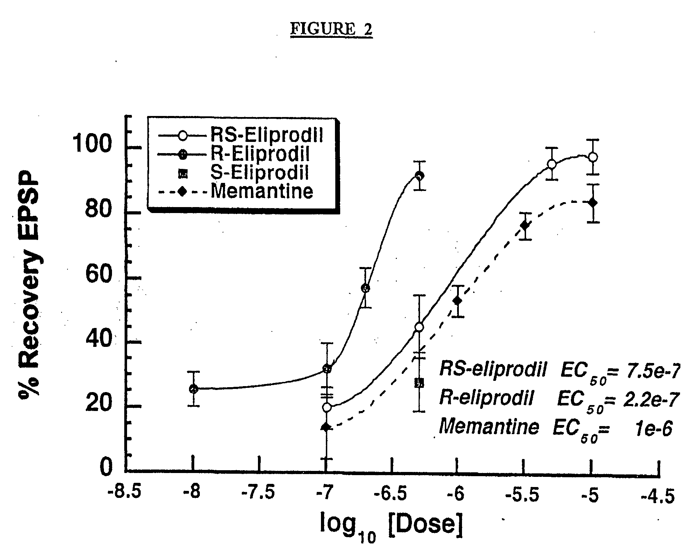

### Dose-response relations comparing the efficacy of Racemic (R/S)-eliprodil (open circles), R-eliprodil (closed circles) and memantine (closed diamonds) for their ability to improve recovery of synaptic transmission following a 15 minute period of hypoxia in hippocampal slices in vitro (Figure 2).

Memantine was selected as it is an NMDA antagonist used clinically.

Preparation of the hypoxic hippocampal slice preparation has been reported by Reyes et al., "Eliprodil, a non-competitive, NR2B-selective NMDA antagonist, protects pyramidal neurons in hippocampal slices from hypoxic/ischemic damage", *Brain Research,* Vol. 782, pp. 212-218, 1998. Briefly, hippocampal slices were prepared acutely the same day as the experiment, and each compound was bath applied 30 minutes prior to delivery of the 15 minute hypoxic insult. Recovery of evoked epsps is expressed as percent of the initial, pre-hypoxia epsp maximum slope, with this percent recovery assessed 60 minutes after reoxygenation. Each point is the mean ± S.E.M. of 8 slices at each dose, and EC₅₀'s were calculated from a single-active site Lineweaver-Burke linear fit. R-eliprodil was statistically significantly more potent (p<0.05, student's t-test) than either racemic-eliprodil, or memantine. At a comparable dose, R-eliprodil is more efficacious than either racemic or S-eliprodil.

R-eliprodil can be administered either systemically or locally with the goal to provide a concentration of R-eliprodil in the retina or optic nerve head of 0.01 to 100µM, preferably 1 to 20µM. Systemic administration means oral, transdermal, subdermal, intraperitioneal, subcutaneous, transnasal, sublingual, or rectal, preferably oral or transdermal. Local administration means, topical ocular, intravitreal, periocular, transcleral, retrobulbar, sub-Tenon, or via an intraocular device, preferably sub-Tenon or retrobulbar or via an intraocular device. Oral delivery of 0.1 - 500 mg/day, preferably 5 -100 mg/day will achieve desired retina concentrations. Regardless of the method of dosing, it is important to maintain a patient's plasma level below 25 ng/ml. This will ensure that untoward QTc effects in the vast majority of the target population are minimal.

It would be advantageous, at the discretion of the skilled clinician, to administer R-eliprodil in combination with one or more other agents for treating glaucoma. Such agents include, but are not limited to, β-blockers (e.g., timolol, betaxolol, levobetaxolol, carteolol, levobunolol, propranolol), carbonic anhydrase inhibitors (e.g., brinzolamide and dorzolamide), α₁ antagonists (e.g. nipradolol), α₂ agonists (e.g., iopidine and brimonidine), miotics (e.g., pilocarpine and epinephrine), prostaglandin analogues (e.g., latanoprost, travaprost, unoprostone, bimatoprost, and compounds set forth in U.S. Patent Nos. 5,889,052; 5,296,504; 5,422,368; 5,688,819; and 5,151,444, "hypotensive lipids" (e.g., compounds set forth in 5,352,708), and other neuroprotectants (e.g., appropriate compounds from WO94/13275, such as, memantine).

### EXAMPLE 3

The following capsules can be made according to the procedures known to those skilled in the art.

| **R-eliprodil Capsules** | |
|---|---|
| **Ingredient** | **mg/Capsule** |
| R-eliprodil hydrochloride | 11.05* |
| Lactose | 201.15 |
| Sodium starch glycolate | 6.6 |
| Magnesium stearate | 2.2 |

| | |
|---|---|
| *Equivalent to 10mg eliprodil as the free base. | |

## Claims

1. Use of R-eliprodil for the manufacture of a medicament for treating glaucoma.

2. Use according to claim 1 wherein said R-eliprodil is formulated as a pharmaceutical composition.

3. Use according to claim 1 or 2 wherein said medicament is in a form suitable for delivery systemically, locally to the eye, orally or transdermally.

4. Use according to claim 3 wherein said medicament is such as to provide an oral dose of 0.1-500mg/day, or 5-100mg/day.

5. Use according to any one of claims 1 to 4 wherein the said medicament further comprises at least one additional agent for treating glaucoma.

6. Use according to claim 5, wherein the additional agent is a β -blocker, a carbonic anhydrase inhibitor, an α₁ antagonist, an α₂ agonist, a miotic, a prostaglandin analogue, a hypotensive lipid, or another neuroprotectant.

## Patentansprüche

1. Verwendung von R-Eliprofil für die Herstellung eines Medikaments für die Behandlung von Glaukom.

2. Verwendung nach Anspruch 1, wobei das R-Eliprofil als pharmazeutische Zusammensetzung formuliert ist.

3. Verwendung nach Anspruch 1 oder 2, wobei das Medikament in einer Form vorliegt, die für die systemische, orale, transdermale Abgabe oder die Abgabe lokal ins Auge geeignet ist.

4. Verwendung nach Anspruch 3, wobei das Medikament so gestaltet ist, dass es eine orale Dosis von 0,1 - 500 mg/Tag oder 5 - 100 mg/Tag bietet.

5. Verwendung nach einem der Ansprüche 1 bis 4, wobei das Medikament des Weiteren mindestens ein zusätzliches Mittel für die Behandlung von Glaukom umfasst.

6. Verwendung nach Anspruch 5, wobei das zusätzliche Mittel ein β-Blocker, ein Carboanhydrasehemmstoff, ein α₁-Antagonist, ein α₂-Antagonist, ein Mitotikum, ein Prostaglandinanalog, ein hypotensives Lipid oder ein anderes Nervenschutzmittel ist.

## Revendications

1. Utilisation de R-éliprodile pour la fabrication d'un médicament traitant le glaucome.

2. Utilisation selon la revendication 1, dans laquelle ledit R-éliprodile est formulé comme composition pharmaceutique.

3. Utilisation selon la revendication 1 ou 2, dans laquelle ledit médicament est sous forme adéquate pour administration systématique, localement dans l'oeil, oralement ou transdermiquement.

4. Utilisation selon la revendication 3, dans laquelle ledit médicament est de nature à procurer une dose orale de 0,1 à 500 mg/jour ou 5 à 100 mg/jour.

5. Utilisation selon l'une quelconque des revendications 1 à 4, dans laquelle ledit médicament comprend en outre au moins un agent supplémentaire pour traiter le glaucome.

6. Utilisation selon la revendication 5, dans laquelle ledit agent supplémentaire est un β-bloquant, un inhibiteur anhydrase carbonique, an antagoniste α₁, un agoniste α₂, un analogue prostaglandine, un lipide hypertenseur ou un autre neuroprotecteur.
